(19) Europäisches Patentamt
European Patent Office
Office européen des brevets

(11) Publication number: **0 114 003**
**A1**

# (12) EUROPEAN PATENT APPLICATION

(21) Application number: **83308031.0**

(22) Date of filing: **30.12.83**

(51) Int. Cl.³: **A 61 K 31/65**, A 61 K 31/71,
A 61 K 45/06
//
(A61K31/65, 31/57),
(A61K31/71, 31/57)

(30) Priority: **31.12.82 GB 8237090**
**14.04.83 GB 8310138**

(43) Date of publication of application: **25.07.84**
**Bulletin 84/30**

(84) Designated Contracting States: **AT BE CH DE FR GB IT LI LU NL SE**

(71) Applicant: **Mortimer, Christopher Harry, Dr., 8 Park Square West Regent Park, London NW1 4LJ (GB)**

(72) Inventor: **Mortimer, Christopher Harry, Dr., 8 Park Square West Regent Park, London NW1 4LJ (GB)**

(74) Representative: **Baverstock, Michael George Douglas et al, BOULT, WADE & TENNANT 27 Furnival Street, London, EC4A 1PQ (GB)**

## (54) Antiandrogen and antibiotic treatment of acne and pharmaceutical preparations therefor.

(57) A pharmaceutical formulation for systemic use in treating acne vulgaris comprising at least one anti-androgen and at least one antibiotic, wherein when the formulation is in unit dosage form the anti-androgen (with progesterone-like properties e.g. cyproterone acetate) is present in an amount of at least 10 mg daily given over 10 days or 100 mg or more in total given during a complete cycle over 21–28 days e.g. about 3.5 mg to 5.0 mg daily or as a single or divided dose.

A calendar pack containing pharmaceutical formulation doses for use in treating female acne vulgaris comprising spaced locations corresponding to the days of a menstrual cycle, dosage forms in a first series of said locations providing at the or each location of the series a daily dose of an anti-androgen and a dose of an antibiotic in pharmaceutically administerable form, and dosage forms at a further series of locations following the first series providing a daily dose of antibiotic in pharmaceutically-administerable form without anti-androgen, wherein the dose of anti-androgen is such that at least 100 mg is provided per cycle.

A topical pharmaceutical formulation for use comprising a combination of at least one anti-androgen and at least one antibiotic, together with at least one of the conventional pharmaceutically-acceptable carriers, diluents and excipents.

EP 0 114 003 A1

0114003

# ANTIANDROGEN AND ANTIBIOTIC TREATMENT OF ACNE AND PHARMACEUTICAL PREPARATIONS THEREFOR

The present invention relates to the treatment of acne vulgaris in male and female subjects.

This condition is in certain cases resistant to conventional treatment with oral antibiotics and topical preparations such as benzoyl peroxide or chlorhexidine. Severe cases may fail to resolve despite years of such conventional treatment and may involve progressive scarring of the face which causes distress in later life when the active phase has abated.

It has now been found that the administration of an antiandrogen, in combination with an antibiotic can produce a very remarkable improvement in severe cases of acne vulgaris in both men and in women and is an effective treatment also in milder cases.

Accordingly, the present invention provides a pharmaceutical formulation for use in treating acne vulgaris by topical or systemic administration which formulation comprises in combination at least one anti-androgen and at least one antibiotic. For systemic administration, the formulation provides for the use of the anti-androgen (with progesterone-like properties e.g. cyproterone acetate) in unit dosage form of an amount of at least 10mg daily given over 10 days or 100mg or more in total given during a complete cycle over 21 - 28 days e.g. about 3.5mg to 5.0mg daily or as a single or divided dose.

An anti-androgen is herein defined as an agent which suppresses the secretion of androgens from either the adrenal glands or testes or ovaries either by suppressing gonadotrophins or ACTH/secretion or reducing androgen secretion directly from the adrenal glands,

ovaries or testes themselves, or is effective locally within the tissues by either displacing androgen from its tissue receptor or competing with its action, or which has opposite actions to androgens (e.g. oestrogen) or impairs the conversion of an androgen substrate to its biologically more active form as testosterone or dihydrotestosterone (e.g. azelaic acid, cimetidine, or 5 alpha reductase inhibitors such as progesterone), or inhibits any other way the synthesis and/or the secretion and/or the action of androgens.

Preferably, the formulations according to this invention include at least one oestrogen e.g ethinyloestradiol or oestradiol benzoate, preferably in combination with at least one non-oestrogenic anti-androgen such as a progesterone-like compound such as medroxyprogestosterone acetate or cyproterone acetate.

Among the non-oestrogenic anti-androgen compounds which may be employed cyproterone acetate is particularly preferred, particularly for formulations other than topical preparations, e.g. oral or injectable (systemic) preparations.

The anti-androgens used are preferably ones which have a substantial effect in decreasing sebum secretion.

For topical preparations, medroxyprogesterone acetate with an oestrogen and the antibiotic is a preferred combination, since cyproterone acetate is not absorbed through the skin.

The present invention includes a pharmaceutical formulation for topical use in treating acne vulgaris in which comprises medroxyprogesterone acetate, at least one oestrogen and at least one antibiotic (with or without retinoic acid/or its derivatives or analogues or salicylic acid).

The invention includes a systemic method for the treatment of acne vulgaris which method comprises administering in combination an anti-androgen, e.g. a combination of cyproterone acetate and at least one oestrogen, and at least one antibiotic.

For oral formulations administration of the antibiotic is preferably at least once per day, more preferably twice daily.

The oral administration of cyproterone acetate and the oestrogen to female patients should however be cyclic, the combination being effective as an oral contraceptive.

Cyproterone acetate may be administered orally for up to 21 days of each cycle depending on dosage. Since cyproterone acetate in larger doses enters the tissues and accumulates producing a depot effect, a single large dose may be sufficient in each cycle.

The oestrogen is preferably administered orally for from 21 to 28 days of the cycle, perferably about 21 days.

Only one oral dose per day is required of the cyproterone acetate and oestrogen but the dose for any day may be split if desired.

Cyproterone acetate is an anti-androgenic compound of the formula:-

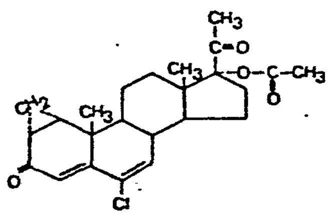

CYPROTERONE ACETATE
(1,2α-methylene-6-chloro-$\Delta^{4,6}$ pregnadiene-17α-ol-3,20-dione-17α-acetate)

It has been used previously in the treatment of men for control of libido in severe hypersexuality and has been proposed for use in treating precocious puberty.

The formulations of the present invention may be designed for oral or parenteral administration. Although the formulations could be for administration by injection, they will most conveniently be for oral administration, e.g. as tablets, capsules or solutions or suspensions.

They may however be formulated as Creams or Lotions for topical administration or as injectable solutions or suspensions for subcutaneous injection or for other forms of injection. They may generally be formulated for any of the know routes of pharmaceutical administration.

Preferably, a formulation for oral administration will contain from 10 to 300mg, preferably about 50mg, of cyproterone acetate per unit dosage form, e.g. per tablet, the suitable daily dose being from 10 to 300mg, preferably 50 to 100mg e.g. 50mg.

The identity of the oestrogen used is not critical. Ethinyloestradiol is preferred. The oestrogen may be present as an anti-androgen or may be provided to provide a contraceptive effect in oral preparations for women since administration of anti-androgens such as cyproterone acetate during pregnancy would be highly undesirable.

Oestrogen has the beneficial effect when administered orally to women of raising plasma sex hormone binding globulin (SHBG) levels thereby diminishing free (biologically active) testosterone and other androgen levels. A suitable dose rate for ethinyloestradiol would be 20ug to 60ug per day orally, preferably 30 to 40ug.

The antibiotic used may be any of those generally known as being suitable for use in treating acne. In particular, the antibiotic may be a tetracycline type antibiotic such as tetracycline itself or chlortetracycline, erythromycin, trimethoprim, sulphamethoxazole, clindamycin, demeclocycline, minocycline or a mixture of two or more of these. Generally, the nature of the

antibiotic is not critical and it may be any antibiotic normally considered suitable for topical application or any antiseptic agent. These antibiotics may of course where appropriate be in the form of their pharmaceutically acceptable salts or other derivatives.

The antibiotic employed may be used in one of the commercially available forms. These include "septrin", trimethaprim and sulphamethoxazole. "Deteclo" is a combination of tetracycline hydrochloride, chlortetracycline hydrochloride and demeclocycline hydrochloride.

Suitable antibiotic contents for unit dosage forms of a composition according to this invention will by way of example be as follows:-

Tetracycline:- 25mg to 2g, more preferably 50mg to 1g,
e.g. 62.5 to 500 mg

Demeclocyline + tetracycline + chlortetracycline:- 3 to 700mg tetracycline, 3 to 700mg chlortetracycline, 18 to 420mg demeclocyline,
or most preferably,

30 to 350mg tetracycline, 30 to 350mg chlortetracycline,

18 to 210mg demeclocycline,

or still more preferably,

60 to 130mg tetracycline, 60 to 130mg chlortetracycline,

and 35 to 140mg demeclocycline.

Clindamycin:- 13mg to 4g, more preferably 25mg to 2g,
eg 25mg to 1.5g

Trimethoprim + sulphamethoxazole:- 20 to 320mg trimethoprim
+ 100mg to 3.2g sulphamethoxazole,
more preferably, 40 to 160mg trimethoprim + 200mg to 1.6g sulphamethoxazole.

Erythromycin:- 250mg to 3g, more preferably 50mg to 1.5g,
eg 65mg to 1g.

Suitable dosage rates for the antibiotic constituent of the treatment of the invention are:-

Erythromycin:- 50mg to 3g per day, preferably 125mg to 1g
per day.

Deteclo:- half to 6 tablets per day preferably 1 to 4
tablets per day.

Clindamycin:- 25mg to 4g per day, more preferably 50mg to
3g per day eg. 150mg per day - preferably in
two equal doses.

Septrin:- half to 8 tablets per day, preferably 1 to 2
tablets per day.

Minocycline hydrochloride (Minocin):- 20 to 500mg per day,
Preferably 25 to 250mg per day.
More preferably 50 to 100mg per day.

It should be understood that any antibiotic generally suitable for treating acne may be used.

In order to facilitate the accurate maintenance of the dosage required when the active components are to be orally administered to female subjects, it is desirable that all of the active ingredients be presented in a calendar pack.

Accordingly, the present invention provides a calendar pack containing pharmaceutical formulations doses for use in treating female acne vulgaris comprising spaced locations corresponding to days of a menstrual cycle, dosage forms in a first series of said locations providing at the or each location of the series a daily dose of an anti-androgen and a dose of antibiotic in pharmaceutically administerable form, and dosage forms at a further series of locations following the first series providing a daily dose of antibiotic in pharmaceutically administerable form without anti-androgen.

More preferably, the invention provides a calendar pack containing pharmaceutical formulation doses for use in treating female acne vulgaris, comprising spaced locations corresponding to days of a menstrual cycle, dosage forms in a first series of said locations

providing at the or each location of the series a daily dose of cyproterone acetate and a daily dose of an oestrogen and a dose of antibiotic in pharmaceutically administerable form and dosage forms at a second series of locations following the first series providing a daily dose of the oestrogen and a dose of antibiotic in pharmaceutically administerable form without cyproterone acetate.

Each dosage location may contain more than one dosage form. For instance, the location for day 1 may contain a formulation in accordance with the invention of cyproterone acetate, oestrogen and antibiotic in a single dosage form. Alternatively, the location for day 1 may contain a dosage form containing cyproterone acetate and oestrogen and a separate dosage from containing antibiotic. These dosage forms may be presented at the 'one days location, one e.g. form for each active ingredient.

An example of a pack according to the invention would be a bubble pack of the conventional kind having a tablet or other dosage form contained in a bubble at each of a series of spaced locations, normally positioned in a closed loop running around the edge of the pack. The locations may be numbered, normally 1 to 28.

A first series of bubbles or other destructible receptacles may contain either one tablet or other dosage form containing all three active ingredients or two or three tablets or other dosage forms, e.g. one containing oestrogen another cyproterone acetate and another antibiotic. Where more than one dosage form is provided they may of course be put in separate bubbles at the same day's location. The first series of locations will preferably correspond to up to the first 21 days e.g. from 7 to 10 days of a 28 day cycle.

The second series of locations may then contain only one dosage form per location containing oestrogen and antibiotic but not cyproterone acetate. The second series may comprise enough locations to bring the user to from the 21st day of a cycle to the 28th day, preferably to about the 21st day.

Further locations may be provided containing a dosage form containing antibiotic but neither of the other active ingredients.

An example of a tablet formulation according to the present invention would for instance be:-

| | | |
|---|---|---|
| 1. | ethinyloestradiol 40µg | 70mg lactose |
| | cyproterone acetate 100mg | 94mg corn starch |
| | 10mg talcum | 3.2mg gelatin |
| | erythromycin 500mg | 2.8mg magnesium stearate |

In order to suppress adrenal androgen production it may be desirable to administer a compound such as prednisolone or dexamethasone which acts as a suppressor for adrenal androgen production. Predisolone is best given in so called reverse rhythm, ie with a higher dose such as 5mg on retiring to suppress the normal early morning rise (3 to 5am) of pituitary ACTH thereby diminishing early morning adrenal gland stimulation. A lesser dose such as 2.5mg taken on waking will then maintain suppression throughout

the morning. Suitable dosages for prednisolone would generally be 3 to 15 mg on retiring and 1 to 5 mg on waking.

If dexamethasone is used, a single dose of from 0.5 to 2 mg on retiring will generally suffice.

Naturally, other adrenal androgen suppressors may be used.

The adrenal androgen production suppressor may be included in the formulations according to the invention. It may be included in the calendar packs described above either as separate dosage forms or in combination with one or none of the other active materials.

The calendar packs provided by the invehtion may provide dosages for each day split between two or more locations each location containing one or more dosage forms, so that the daily dose of the cyproterone acetate, oestrogen antibiotic and optionally suppressor may be split as desired for e.g. morning and night doses. Not all of the ingredients need be present in any one dose.

Other agents known for use in the treatment of acne may be included in formulations according to the present invention. For instance, 13-cis retinoic acid may be included, suitably in amounts sufficient to provide a dose of about 2.0 mg per kg body weight of the patient to be treated. For instance, a preparation to be taken twice daily might contain from 50 to 80 mg of 13-cis retinoic acid. This compound appears to have a direct inhibitory

effect on sebum secretion. Also Zinc as Zinc Sulphate up to 250mg of elemental zinc daily in divided doses orally may be combined with the treatment.

Other compounds used in the treatment of acne which may be included are retinoic acid (as Retin A or other retinoids) Vitamin A or its derivatives or analogues or salicylic acid or its salts. Examples of topical preparations according to the invention are as follows:

| | | |
|---|---|---|
| 1 a. | Clindamycin | 0.5% - 5.0% |
| | Medroxyprogesterone Acetate | 0.1% - 1.0% |
| | Oestradiol Benzoate | 0.001% up to 1.0% |
| | Retinoic Acid | 0.0125% - 0.4% |
| | Salicylic acid | 0.1% - 5.0% |
| 1 b. | Clindamycin | 0.5% - 5.0% |
| | Medroxyprogesterone Acetate | 0.1% - 1.0% |
| | Oestradiol Benzoate | 0.001% up to 1.0% |
| | Retinoic Acid | 0.0125% - 0.4% |
| 1 c. | Clindamycin | 0.5% - 5.0% |
| | Medroxyprogesterone Acetate | 0.1% - 1.0% |
| | Oestradiol Benzoate | 0.001% up to 1.0% |
| 1 d. | Clindamycin | 0.5% - 5.0% |
| | Medroxyprogesterone Acetate | 0.1% - 1.0% |
| 1 e. | Clindamycin | 0.5% - 5.0% |
| | Medroxyprogesterone Acetate | 0.1% - 1.0% |
| | Salicylic Acid | 0.1% - 5.0% |

| | | |
|---|---|---|
| 1 f. | Clindamycin | 0.5% - 5.0% |
| | Medroxyprogesterone Acetate | 0.1% - 1.0% |
| | Salicylic Acid | 0.1% - 5.0% |
| | Retinoic Acid | 0.0125% - 0.4% |

or:

g. Any combination of any two ingredients.

It should be understood that any antibiotic generally suitable for treating acne may be used in a topical dose range 0.5% - 5.0%.

Each of the above could be made up either as an ointment, in which case the balance of each would be an ointment base such as "unguentum Merck", or as a lotion using an appropriate lotion base. Lotions are suitably applied to the entire affected area whilst creams may be applied to particularly large lesions to maintain contact through the day.

Treatment may involve the simultaneous use of both oral and topical preparations according to the invention. Thus the treatment might suitably include the cyclical administration of oral preparations using calendar packs as described above and continuous use of a topical preparation.

SYSTEMIC PREPARATIONS

Clinical Examples

a) Severe pustular/cystic acne with extensive previous scarring

Two female patients aged 17 years and 26 years had a history of severe pustular and cystic acne of the face, chest and shoulders both for five years previously despite long term antibiotic therapy.

In both patients there was evidence of extensive scarring of the tissues involved. Both patients had noted scalp hair loss and one had mild facial hirsuties and both had irregular periods prior to therapy. In order to effect resolution of the infected areas as rapidly as possible in these patients who were greatly distressed by their appearance, and to prevent further scarring treatment was begun with the following combination of drugs:

Daily therapy:

Prednisolone 5.0mg nocte; 2.5mg mane

Deteclo (Chlortetracycline HCl 115.4mg; Tetracycline HCl 115.4mg; Demeclocycline HCl 69.2mg) one nocte and mane.

Cyclical therapy:

Ethinyl oestradiol 40ug mane daily for 25 days

Cyproterone acetate 50mg mane and 50mg nocte for 21 days

Following the "period" the cyclical therapy was restarted. One patient continued this pattern of treatment for four months. The number of days per month of cyproterone acetate treatment was then reduced to 15 for 4 months and to 10 for the remaining period of treatment.

The dosage of ethinyl oestradiol was reduced to 30μg per day after nine months. The morning dose of prednisolone was stopped after five months and prednisolone treatment ended after seven months. In the other patient, the daily therapy was maintained for 10 months when the prednisolone was reduced to 5mg nocte for three months and then stopped completely. After 12 months the Deteclo was changed to Erythromycin stearate 250mg mane and nocte. The cyclical therapy continued as above for four months when cyproterone acetate was reduced to 50mg mane and nocte for the first 15 days of the cycle and then after a further eight months to 50mg mane and nocte for 10 days only. After three months of such treatment the cyproterone acetate was stopped and the patient continued with ethinyl oestradiol 30ug daily for 21 days together with medroxyprogesterone acetate 10mg daily from days 16-21 of the cycle together with erythromycin 250mg mane and nocte only. Within six months the patient's acne had returned although not as severe as previously and therefore treatment with ethinyl oestradiol 40ug for 21 days together with cyproterone acetate 50mg twice daily for the first 7 days of each cycle was restarted. This resulted in complete clearing of the acne in two months.

In both female patients the initial combination of cyclical/ anti androgen (cyproterone acetate and ethinyl oestradiol) and antibiotic therapy together with reverse rhythm prednisolone resulted in rapid improvement of the patients' acne on the face, chest, shoulders and back with a substantial clearing within 6 to 8 weeks and complete resolution of the active lesions at five months and at 10 months. In both patients hair fall was reduced after 6-8 weeks with regrowth noticeable after 6-7 months. The patient with facial hirsuties noted an improvement after 7 months. In one patient at 12 months dermabrasion was carried out to the deeply scarred but now inactive areas, with good effect and continued cosmetic improvement over the subsequent six months. At thirty months of therapy the patient still retained clear skin. During therapy both patients noted regular but light withdrawal bleeds 4-7 days after stopping the ethinyl oestradiol. After 24 and 30 months cervical smears were normal and there were no significant changes in blood pressure and clinical examination was otherwise normal. Prior to therapy one patient was within the ideal range for weight 58.3kg (ideal 49-59) while the other was below this 57.5kg (ideal 62-69).

During treatment their maximum weights were 62.4 and 65.5kg respectively.

b) <u>Severe pustular/cystic acne with limited previous scarring</u>

Five female patients were in this group with involvement of the face, chest, shoulders and back aged 16-30 years with a history of resistant acne for 3-15 years. The patients received the following treatment for 4-15 months.

<u>Daily therapy</u>

Deteclo one mane and one nocte; or erythromycin stearate 250mg mane and nocte.

<u>Cyclical therapy</u>

Ethinyl oestradiol 40μg mane for 21 days

Cyproterone acetate 50mg mane and nocte for the first ten days of the cycle.

Treatment was restarted following the "period".

In all patients within four weeks there had been an improvement in the skin lesions. Within six months all patients had clearing of the lesions on the body and by 6-12 months complete resolution of the facial lesions. In the first 1-3 weeks two patients noted an increase in facial lesions. In one of them additional new lesions appeared over the front of the neck.

In both patients complete resolution of these treatment induced lesions occured within 3-6 weeks on the same therapy. All patients experienced lighter "periods" beginning 4-7 days after stopping ethinyl oestradiol. Two patients noted an unwanted increase in body weight together with an increase in breast size. Their basal weights were 50kg (ideal 47-53) and 49.9kg (ideal 45-49) increasing to a maximum of 57kg at 7 months and 54kg at 6 months respectively. In both patients resolution of their skin lesions had resulted in a more cheerful disposition and increase in their food consumption as a result of an enhanced social life. One patient was started on Apisate (diethylpropion HCl 75mg, thiamine HCl 5mg, riboflavine 4mg, pyridoxine HCl 2mg, nicotinamide 30mg) with successful appetite suppression and weight loss. The other patient despite an attempt to diet did not manage to lose weight. Two patients with hair loss noted a reduction in hair fall after 2-4 months of therapy and improved growth thereafter.

c) Moderate acne with minimal scarring

One female patient 19 years with moderate facial acne without scarring which had failed to respond to antibiotic therapy alone over one year was started on cyclical anti androgen treatment with erythromycin as in b)

with complete resolution within 3 months. Her weight remained unchanged.

d) Mild acne

Two female patients with mild facial acne 17 and 20 years (the latter with gross hirsuties) of 1-7 years duration and resistant to antibiotic therapy alone were treated with cyclical anti androgen treatment and erythromycin as in b).

There was complete resolution of the lesions within 1-2 months. After six months therapy in the hirsute patient there was a marked reduction in the rate of hair growth on the face and body with the replacement of coarse dark hairs with finer lighter coloured hairs. Neither patient had any significant change in weight or breast size.

e) Effect of reducing anti-androgen dosage

Initially, female patients were treated with Ethinyloestradiol 40ug daily for 21 days combined with Cyproterone Acetate 100mg as 50mg twice daily for the first 10 days of each cycle combined with either Deteclo one b.d. or Erythromycin 250mg twice daily. Subsequently, as the acne has cleared the amount of Cyproterone Acetate was reduced.

Patient No 1: Aged 20 years on starting treatment had been resistant to 1G of Erythromycin daily for a minimum of one year before. Within 4 months the acne was completely cleared from the face. She continued with

the full dosage for 2 years and then she continued with:
Ethinyloestradiol 40ug daily for 21 days of each cycle.
Cyproterone Acetate 50mg daily for the first 10 days of each cycle.
Erythromycin 250mg b.d.
She continued on the above treatment without the return of her acne for 2 months when the dose was reduced to:
Ethinyloestradiol 40ug daily for 21 days of each cycle,
Cyproterone Acetate 25mg daily for the first 10 days of each cycle.
Erythromycin 250mg b.d.
Again she remained clear of acne so that one month later she continued with:
Ethinyloestradiol 40ug daily for 21 days of each cycle,
Cyproterone Acetate 25mg alternate days over 10 days (i.e. on 5 occasions equivalent to 125mg)
Erythromycin 250mg b.d.

2 months later the patient had the return of her first acne lesion, a small pustule to the left side of the chin, the first for approximately one year.

Patient No 2: This patient was aged 30 years when she began with the following treatment:
Ethinyloestradiol 40ug daily for 21 days of each cycle.
Cyproterone Acetate 100mg daily for the first 10 days of each cycle. (As 50mg b.d.)
Deteclo one tablet b.d.

She continued on the above regimen with complete clearing of the pustular lesions and in particular hard painful lesions around the chin at 6 months such that after a further 2 months she continued with:

Ethinyloestradiol 40ug daily for 21 days of each cycle.

Cyproterone Acetate 100/50mg alternate days over 10 days.

Deteclo one tablet b.d.

After 2 months she then continued with:

Ethinyloestradiol 40ug daily for 21 days of each cycle.

Cyproterone Acetate 50mg daily for 10 days.

Erythromycin 250mg b.d.

The following month the patients skin was still completely clear, and so she continued with the same dose for a further five months. Then she continued with:

Ethinyloestradiol 40ug daily for 21 days of each cycle.

Cyproterone Acetate 50mg daily for 7 days.

Erythromycin 250mg b.d.

On this her skin remained completely clear, and so after a further four months she continued with:

Ethinyloestradiol 40ug daily for 21 days of each cycle.

Cyproterone Acetate 25mg daily for 7 days (i.e. 175mg per cycle).

Erythromycin 250mg b.d.

The next month she had her first acne lesion for

approximately one year. The patient then continued with:

Ethinyloestradiol 40ug daily for 21 days of each cycle.

Cyproterone Acetate 25mg alternate days of 4 occasions. (i.e. 100mg per cycle).

Erythromycin 250mg b.d.

Two months later there was worsening of the acne in that the previous hard painful lumps around the chin were beginning to return. The patient was asked to continue with:

Ethinyloestradiol 30ug daily for 21 days of each cycle.

Cyproterone Acetate 25mg on alternate days on 3 occasions (i.e. 75mg per cycle).

Erythromycin 250mg b.d.

When the patient was seen the following month the acne was clearly worsening and she noted that her hair was beginning to fall again as it was when she was first seen.

From this study the following points emerge:

1. Deteclo one b.d. or Erythromycin 250mg b.d. appear to be of equivalent effect. However, Erythromycin is preferable in view of the photosensitivity which may occur with Tetracyline contained in Deteclo.

2. The first patient's acne had failed to clear with Erythromycin 1G a day for the previous year. However, when this dose was reduced to 250mg b.d. (500mg per day) and combined with Ethinyloestradiol and Cyproterone Acetate the acne cleared completely in 4 months. The

addition of cyclical antiandrogen therapy therefore appears to allow the dose of antibiotics to be reduced and for a beneficial response to be seen with complete clearing of acne despite the reduced dose of antibiotics. By a synergistic action the inclusion of an antibiotic allows a lower dose of antiandrogen to be used which is highly desirable in clinical practice.

3. In both of the above patients as the total dose of Cyproterone Acetate per cycle was reduced to 125mg in the first patient and 175mg in the second patient the acne began to return.

TOPICAL PREPARATIONS

Clinical examples

f) A female patient aged 18 years had facial acne and also some lesions over the back for the last 5 years beginning at puberty. The patient had not been without acne during this time despite various topical preparations being suggested to her or which she obtained herself from the pharmacy.

The patients treatment regimen was as follows:
Wash face with a neutral soft soap, dry skin gently, and apply over all the lesions a lotion of formulation 1(a) above with Clindamycin at 1.5%, Medroxyprogesterone Acetate at 0.1%, Oestradiol Benzoate at 0.1%, Retinoic Acid at 0.05%, Salicylic Acid at 0.1%. To individual, raised or painful lesions a cream was applied of formulation 1(c) above with Clindamycin at 1.5%, Medroxyprogesterone Acetate at 0.1% and Oestradiol Benzoate at 0.1% in Ung Merck.

The applications were carried out in the morning and in the evening and the amount used in each application

was generally between 1 and 2 ml. At two weeks the lesions on the patient's back cleared and did not return although the topical applications were discontinued.

The patient applied the lotion and cream initially to the right side of the face only. After 4 weeks there was some clearing on the right extending also across to the left side of the midline.

At nine weeks there were no active lesions on the face nor back.

During the 9 weeks that the patient had been using the applications to the face, there were no ill effects, the menstrual cycle was not disturbed and there was clear evidence of healing.

Subsequently, on stopping the twice daily applications the patient's face and back remained clear for 4 weeks. After 5 weeks and 4 days without treatment, a few acne lesions began to appear around the chin and at the angles of the nose and on the forehead. These were in the central area previously affected, although at this time just discernible under the skin. At the same time the acne had clearly returned to the back and had been present for 2 days only. The patient therefore continued with the treatment as follows:

FORMULATION "C" - 2 Times daily

Clindamycin 1.5%

Medroxyprogesterone Acetate 0.1%

Oestradiol Benzoate 0.01%

Salicylic acid 0.1%

(Retinoic Acid 0.05% was omitted from this preparation)

After one week the acne again began to clear and there were no active lesions remaining on the face. On the back, approximately 40% of the previous spots were healed completely. 30% were still active and the rest were of intermediate severity.

During the following week the patient continued with:

FORMULATION "S" - 3 Times daily

Erythromycin 1.5%

Medroxyprogesterone Acetate 0.5%

Oestradiol Benzoate 0.02%

Salicylic Acid 2%

The patient continued with this for one week and all the remaining acne lesions on the face cleared. The back was clearing also with fading of the lesions. She then continued on FORMULATION "S" six times a day to the face and back. There was no peeling with this regimen and no undue dryness. The face remained completely clear and on the back the lesions were fading. The following week the patient continued on FORMULATION "S" again six times daily. The face remained clear and the back continued to clear.

The following week the patient started with:

FORMULATION "P" - 3 Times daily

| | |
|---|---|
| Erythromycin | 1.5% |
| Progesterone | 2.5% |
| Oestradiol Benzoate | 0.01% |
| Salicylic Acid | 3.0% |

The patients face was completely clear. The back was continuining to clear with rapid fading now of the lesions. There was some peeling of the skin around the chin and on the back.

The patient then continued for a week with:

FORMULATION "PK" - 3 Times daily

| | |
|---|---|
| Erythromycin | 0.75% |
| Progesterone | 1.25% |
| Oestradiol Benzoate | 0.005% |
| Salicylic Acid | 1.5% |
| Retinoic Acid | 0.0125% |

The skin on both face and back was now completely clear i.e. 8 weeks after re-appearance of the acne. The patient continued to date using FORMULATION "PK" once per day with the skin of both face and back still completely clear.

Topical preparations (both Lotions and Creams) according to the invention are also efective in the treatment of male acne.

Clinical Examples

(g) A male patient aged 20 years had severe pustular acne and greasy skin since the age of 13-14 years. Previously he used topical preparations from the chemists, using items such as Clearasil, also benzoyl peroxide, and Retin A, combined with oral tetracycline 500mg b.d. for five years. More recently he used benzoyl peroxide 5% gel together with two septrin tablets b.d. None of these medications was effective. All were stopped.

He therefore started started using the topical preparations of the present invention as for female patients using the Lotion, Clindamycin 1.5%, Medroxyprogesterone Acetate 0.1%, Oestradiol Benzoate 0.1%, Retinoic Acid 0.05%, Salicylic Acid 0.1% for wide application; the cream Clindamycin 1.5%, Medroxy- progesterone Acetate 0.1%, Oestradiol Benzoate 0.1% to localised lesions, together with a neutral cream soap. He also used a polytar shampoo and hair conditioner.

The patient applied the Lotion and Cream to the right side of the face only for one week and then subsequently to both sides. After six weeks there had been a marked improvement in the skin in that the pustular lesions were actively resolving and the patient noted a rapid decrease (within hours of the application) in the tenderness previously associated with his lesions. The patient also had pustular acne over the back and shoulders and again at six weeks this was resolving.

After 17 weeks of the original formulation he continued with a preparation omitting the retinoic acid. The patient used this once per day only or on some instances only 3-4 times per week. After a total of 25 weeks of treatment, 8 weeks of treatment without retinoic acid his shoulders were completely clear and his face clearing still further. There were no significant changes in the haematology, biochemistry or endocrine/sperm count profiles nor in potency.

h) A male patient aged 18 years with severe cystic acne from the age of 15 years affecting predominantly the back. He had received Tetracycline 1G per day or Septrin 2 tablets b.d. combined with a variety of skin washes. Recently he had been receiving Erythromycin 1G per day together with Prednisolone and had returned from Bermuda where he had been sunbathing in an effort to produce resolution of his acne. This had been supplemented also in the previous year by ultra-violet light from a sunbed on a regular basis. It was clearly evident that these forms of treatment were ineffective.

Therefore, he was started on the present topical preparations, Cream and Lotion as in the previous male patient, applied twice daily to the back. Within 12 weeks there had been a great improvement with a reduction in the number of active lesions. By 26 weeks there were no active lesions remaining on the back. The previously active pustules were replaced by pink blemishes in the process of healing. By the end of 26 weeks there had been no significant change in the haematology, biochemistry or endocrine/sperm count profiles nor in potency.

i) A male patient aged 15 years had recurrent pustular acne with comedones present since the age of 12 years. Previously he had used topical preparations from the Pharmacy without effect. He was started on the topical preparation, Lotion only, containing:

Formulation ST:

Clindamycin 1.5%
Medroxyprogesterone Acetate 0.5%
Oestradiol Benzoate 0.1%
Retinoic Acid 0.2%
Salicylic Acid 0.1%

The patient applied the Lotion over the complete face, forehead, chin and sides of face which were affected by acne.

After 3 weeks there was a great improvement in the patients acne with clear evidence of resolution and after 9½ weeks the skin was clear of active lesions. The patient then continued to apply Formulation ST to the left side of the face only. After a further 5 weeks, acne again appeared on the right side of the chin with some lesions on the forehead. The left side of the face remained clear.

The treatment was without side effects apart from some dryness to the left nasal fold and left side of the nose after 14½ weeks of continuous treatment. This dryness disappeared on using the preparation once daily without the return of the acne.

The use of oral or other systemic modes of administration of anti-androgens will not be suitable for male patients in general because of undesired effects of the hormonal constituent of the formulations. However, there may be special cases in which such administration routes would benefit a male patient. The formulations, according to the invention should not include any active ingredients incompatible with the treatment of acne or intended for treatment of wholly different conditions. For the sake of illustration, the inclusion of, for instance, cytotoxic drugs, such as cytidine derivatives, would not be within the scope of the invention. A formulation containing such drugs would not be suitable for use in treating acne.

Further clinical trials have established that the omission of the antibiotic from the formulations of the present invention results in incomplete clearing of the acne.

CLAIMS

1. A pharmaceutical formulation for use in treating acne vulgaris comprising at least one anti-androgen and at least one antibiotic, wherein when the formulation is in unitdosage form the anti-androgen is present in an amount of at least 10mg.

2. A formulation as claimed in claim 1 further comprising a pharmaceutically-acceptable carrier, diluent or excipent.

3. A formulation as claimed in claim 1 or claim 2, containing as antibiotic a tetracycline-type antibiotic, erythromycin, trimethoprim, sulphamethoxazole, minocycline, clindamycin, or demeclocycline, or a mixture of two or more thereof.

4. A formulation as claimed in any preceding claim comprising an oestrogen.

5. A formulation as claimed in claim 4 wherein the oestrogen is ethinyloestradiol or oestradiol benzoate.

6. A formulation as claimed in any preceding claim comprising cyproterone acetate or medroxyprogesterone acetate or a pharmaceutically-equivalent agent.

7. A formulation as claimed in any one of claims 1 to 3 comprising an oestrogen and cyproterone acetate as anti-androgens.

8. A formulation as claimed in any preceding claim in the form of a unit dosage form for oral administration.

9. A formulation as claimed in claim 8 which provides from 10 to 300 mg of cyproterone acetate per unit dosage form.

10. A formulation as claimed in claim 9 which provides about 50 mg of cyproterone acetate per unit dosage form.

11. A formulation as claimed in claim 5 in unit dosage form which provides from 20 to 60 µg per unit dosage form of ethinyloestradiol or its pharmacologically-active equivalent.

12. A formulation as claimed in any one of claims 8 to 11, containing from 25 mg to 2g of tetracycline.

13. A formulation as claimed in claim 12 containing from 50mg to 1g of tetracycline.

14. A formulation as claimed in claim 13 containing from 62.5 to 500 mg of tetracycline.

15. A formulation as claimed in any one of claims 8 to 11 containing a mixture of tetracycline, chlortetracycline and demeclocycline.

16. A formulation as claimed in claim 15 containing from 3 to 700 mg tetracycline, from 3 to 700 mg chlortetracycline, and from 18 to 420 mg of demeclocycline.

17. A formulation as claimed in claim 16 containing from 60 to 130 mg tetracycline, 60 to 130 mg chlortetracycline and 35 to 140 mg demeclocycline.

18. A formulation as claimed in any one of claims 8 to 11 containing from 13 mg to 4g of clindamycin.

19. A formulation as claimed in claim 18 containing from 25 mg to 2g of clindamycin.

20. A formulation as claimed in claim 19 containing from 25 mg to 1.5g of clindamycin.

21. A formulation as claimed in any one of claims 8 to 11 containing a mixture of trimethoprim and sulphamethoxazole.

22. A formulation as claimed in claim 21 containing from 20 mg to 320 mg of trimethoprim and 100 mg to 3.2g of sulphamethoxazole.

23. A formulation as claimed in claim 22 containing from 40 to 160 mg of trimethoprim and 200mg to 1.6g of sulphamethoxazole.

24. A formulation as claimed in any one of claims 8 to 11 containing from 20 to 500 mg of minocycline.

25. A formulation as claimed in claim 24 containing from 25 to 250 mg of minocycline.

26. A formulation as claimed in claim 25 containing 50 to 100 mg of minocycline.

27. A formulation as claimed in any one of claims 8 to 11 containing from 25 mg to 3g of erythromycin.

28. A formulation as claimed in claim 27 containing from 50 mg to 1.5g of erythromycin.

29. A formulation as claimed in claim 28 containing from 125 mg to 1g of erythromycin.

30. A formulation as claimed in any one of claims 2 to 7 in a form suitable for topical administration.

31. A formulation as claimed in claim 2 to 7 in the form of a cream, ointment or lotion.

32. A formulation as claimed in any one of claims 2 to 7 in a form suitable for parenteral administration.

33. A formulation as claimed in any preceding claim further comprising an adrenal androgen production suppressing agent.

34. A formulation as claimed in claim 33 wherein the suppressant is prednisolone or dexamethasone.

35. A calendar pack containing pharmaceutical formulation doses for use in treating female acne vulgaris comprising spaced locations corresponding to days of a menstrual cycle, dosage forms in a first series of said locations providing at the or each location of the series a daily dose of an anti-androgen and a dose of an antibiotic in pharmaceutically administerable form, and dosage forms at a further series of locations following the first series providing a daily dose of antibiotic in pharmaceutically-administerable form without anti-androgen, wherein the daily dose of anti-androgen is such that at least 100mg is provided per cycle.

36. A calendar pack containing pharmaceutical formulation doses for use in treating female acne vulgaris comprising spaced locations corresponding to days of a menstrual cycle, dosage forms in a first series of said locations providing at the or each location of the series a daily dose of cyproterone acetate and a daily dose of an oestrogen and a dose of antibiotic in pharmaceutically administerable form and dosage forms at a second series of locations following the first series providing a daily dose of the oestrogen and a dose of antibiotic in pharmaceutically-administerable form without cyproterone acetate, wherein the daily dose of anti-androgen is such that at least 100mg is provided per cycle.

37. A pack as claimed in claim 36 wherein the oestrogen is ethinyloestradiol.

38. A pack as claimed in claim 37 wherein the daily dose of oestrogen provided is 20 to 60 µg of ethinyloestradiol.

39. A pack as claimed in claim 38 wherein the daily dose of oestrogen provided is about 40 µg of ethinyloestradiol.

40. A pack as claimed in any one of claims 36 to 39 wherein the or each location of the first series contain separate dosage forms providing cyproterone acetate, oestrogen and antibiotic respectively.

41. A pack as claimed in any one of claims 36 to 40 wherein the or each location of the first series contains a dosage form providing a mixture of cyproterone acetate, oestrogen and antibiotic.

42. A pack as claimed in any one of claims 35 to 41 wherein the location or locations in the first series correspond to up to the first 21 days of a menstrual cycle.

43. A pack as claimed in claim 42 wherein the location or locations in the first series correspond to about the first 10 days of a menstrual cycle.

44. A pack as claimed in any one of claims 35 to 43 wherein the locations in the first and second series together correspond to about the first twenty one days of a menstrual cycle.

45. A pack as claimed in any one of claims 36 to 44 wherein the or each location in the first series contains a dose of from10 to 300 mg of cyproterone acetate.

46. A pack as claimed in claim 45 wherein the or each location in the first series contains a dose of from 50 to 100 mg of cyproterone acetate.

47. A pack as claimed in any one of claims 36 to 46 wherein dosage forms containing antibiotic in the absence of oestrogen or cyproterone acetate are provided at a third series of locations following the second series.

48. A pack as claimed in any one of claims 36 to 47 wherein the antibiotic content of the dosage forms of the first and second or first, second and,if provided,third series of locations is as defined in any one of claims 12 to 29.

49. A pack as claimed in any one of claims 35 to 48 wherein each dosage form is a tablet or capsule.

0114003

European Patent Office

# EUROPEAN SEARCH REPORT

Application number

EP 83 30 8031

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| X | UNLISTED DRUGS, vol. 16, no. 8, August 1964, page 58, Chatham, New Jersey, USA<br>* Page 58 o, "Promone" *<br>--- | 1,2,6 | A 61 K 31/65<br>A 61 K 31/71<br>A 61 K 45/06 //<br>(A 61 K 31/65<br>A 61 K 31/57 )<br>(A 61 K 31/71<br>A 61 K 31/57 ) |
| A | UNLISTED DRUGS, vol. 26, no. 6, June 1974, page 83, Chatham, New Jersey, USA<br>* Page 83 j, "SH 81041" *<br>--- | 1-49 | |
| A | UNLISTED DRUGS, vol. 23, no. 2, February 1971, page 27, Chatham, New Jersey, USA<br>* Page 27 m, "Verafem" *<br>--- | 1-49 | |
| A | ROTE LISTE, 1974, Editio Cantor, Aulendorf/Württ., DE.<br>* No. 32 158 B, no. 32 159 B "Cortidexason"; no. 72 067 B "Prednitracin" *<br>--- | 1-49 | TECHNICAL FIELDS SEARCHED (Int. Cl. 3)<br>A 61 K |
| A | US-A-4 018 918 (DONALD E. AYER)<br>* Column 30, lines 30-38 *<br>--- | 1-49 | |
| A | FR-M- 3 526 (CHARLES GRUPPER)<br>* Page 1, left-hand column *<br>----- | 1-49 | |

The present search report has been drawn up for all claims

| Place of search | Date of completion of the search | Examiner |
|---|---|---|
| THE HAGUE | 09-04-1984 | BRINKMANN C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82